# EUROPEAN PATENT APPLICATION

(11) **EP 4 238 996 A2**
(43) Date of publication of application: **06.09.2023**
(21) Application number: 21919891.8
(22) Date of filing: 08.12.2021
(51) Int. Cl.: C07K 16/40, A61P 31/12, A61K 39/00

(54) **ANTIVIRAL ANTIBODY AGAINST SARS-COV2 BINDING TO ACE2, AND USE THEREOF**

(30) Priority: 13.01.2021 KR 20210004359; 01.10.2021 KR 20210130808
(71) Applicant: Daegu Gyeongbuk Institute Of Science and Technology, Daegu 42988 (KR)
(72) Inventor: YEA, Kyung Moo, Dalseong-gun Daegu 43016 (KR); PARK, Jun Kook, Gwangmyeong-si Gyeonggi-do 14246 (KR)
(74) Representative: Roos, Peter
(86) International application number: PCT/KR2021/018555
(87) International publication number: WO 2022/154267

(57) **Abstract**

The present invention relates to an antiviral antibody against SARS-CoV2 binding to ACE2 and a use thereof. The objective of the present invention is to prepare an antibody which binds to hACE2, and confirm that the antibody exhibits an antiviral effect against SARS-CoV2 by verifying that each antibody inhibits SARS-CoV2 virus entry into cells, thereby providing a composition comprising the antibody as a pharmaceutical composition for preventing or treating infection caused by SARS-CoV2.

## Description

### Technical Field

The present disclosure relates to an antiviral antibody against SARS-CoV2 binding to ACE2, and a use thereof.

### Background Art

Coronavirus is a virus consisting of single fragments of (+) strand RNA genome with a size of about 27-32 kb and distributed in humans and other mammals. Coronavirus is known to produce 6∼8 subgenomic RNAs with genome-RNA and common mRNA at 3' terminal by undergoing replication and transcription. Although most people experience only mild symptoms, coronavirus infection is highly infectious, with more than 10,000 people infected with severe acute respiratory syndrome (SARS, 10% fatality) coronavirus and Middle East respiratory syndrome (MERS, 37% fatality) coronavirus in the past 20 years.

COVID-19, a recently discovered new coronavirus (severe acute respiratory syndrome coronavirus 2, SARS-CoV-2) infection, is an acute respiratory syndrome that was found in China on December 1, 2019 and first reported on December 12, 2019, with symptoms such as fever, cough, dyspnea, and atypical pneumonia. Since January 2020, it spread widely outside China and is rapidly spreading at an alarming level before and after the Lunar New Year holiday in China, causing a surge in infections and crippling of the entire city of Wuhan.,

SARS-CoV2 binds to an angiotensin-converting enzyme 2 (hACE2) protein which is mainly present in human lung epithelial cells and invades into cells to replicate the viral genetic material in human cells and reproduce the virus. A fundamental way to treat COVID-19 infection is to find strategies to prevent the corona virus from entering human cells. The spike protein S 1-receptor binding domain (RBD) present on the surface of corona virus binds to a hACE2 protein, wherein the 3D bound structure has been revealed by recent studies.

It is expected that blocking of the binding between SARS-CoV2 and hACE2 may fundamentally prevent cellular invasion of coronaviruses, and there are studies continued on novel means capable of blocking the binding between SARS-CoV2 and hACE2 in an attempt to prevent or treat infection by SARS-CoV2, COVID-19.

### Disclosure of the Invention

### Technical Goals

In order to solve the above problem, an object of the present disclosure is to prepare an antibody binding to hACE2 and provide a composition including the antibody as a pharmaceutical composition for preventing or treating infection by SARS-CoV2, by proving that each antibody inhibits the entry of SARS-CoV2 virus into cells so as to determine that the antibody exhibits an antiviral effect against SARS-CoV2.

### Technical Solutions

The present disclosure provides an antiviral antibody against SARS-CoV2 binding to ACE2.

In addition, the present disclosure provides a pharmaceutical composition for preventing or treating infection by SARS-CoV2, including the antibody as an active ingredient.

### Advantageous Effects

According to the present disclosure, the antibodies of the present disclosure bind to hACE2 and inhibit the entry of SARS-CoV2 virus into cells, thereby exhibiting an effect of preventing or treating infection by SARS-CoV2 virus.

### Brief Description of Drawings

FIG. 1 shows results of evaluating the binding of antibodies of the present disclosure to hACE2.
FIG. 2 shows a result of evaluating whether antibodies of the present disclosure exhibit an antiviral effect against SARS-CoV2.

### Best Mode for Carrying Out the Invention

The terms used herein have been selected from currently widely used general terms as much as possible in consideration of functions herein, but these may vary depending on the intentions or precedents of those skilled in the art, the emergence of new technologies, and the like. In addition, in specific cases, there are terms arbitrarily selected by the applicant, and in this case, the meaning will be described in detail in the description of the disclosure. Therefore, the terms used herein should not be defined as simple names of terms, but based on the meaning of the term and the overall contents of the present disclosure.

Unless otherwise defined, all terms used herein, including technical or scientific terms, have the same meaning as commonly understood by those skilled in the art to which the present disclosure pertains. Terms such as those defined in commonly used dictionaries should be construed as having meanings consistent with the meaning in the context of the relevant art and are not to be construed in an ideal or overly formal meaning unless clearly defined in the present application.

The numerical range includes the numerical value defined in the above range. All maximum numerical limits given herein include all lower numerical limits as clearly stated on the lower numerical limits. All minimum numerical limits given herein include all higher numerical limits as clearly stated on the higher numerical limits. All numerical limits given herein will include all better numerical ranges within a wider numerical range as clearly stated on narrower numerical limits.

Hereinafter, the present disclosure will be described in more detail.

The present disclosure provides an antiviral antibody against SARS-CoV2 binding to ACE2.

The antibody may include a light chain having an amino acid sequence represented by SEQ ID NO: 1; and a heavy chain having an amino acid sequence represented by SEQ ID NO: 2, or a light chain having an amino acid sequence represented by SEQ ID NO: 3; and a heavy chain having an amino acid sequence represented by SEQ ID NO: 4, or a light chain having an amino acid sequence represented by SEQ ID NO: 5; and a heavy chain having an amino acid sequence represented by SEQ ID NO: 6, or a light chain having an amino acid sequence represented by SEQ ID NO: 7; and a heavy chain having an amino acid sequence represented by SEQ ID NO: 8, or a light chain having an amino acid sequence represented by SEQ ID NO: 9; and a heavy chain having an amino acid sequence represented by SEQ ID NO: 10, or a light chain having an amino acid sequence represented by SEQ ID NO: 11; and a heavy chain having an amino acid sequence represented by SEQ ID NO: 12.

In addition, the light chain having the amino acid sequence represented by SEQ ID NO: 1 may be encoded by a sequence represented by SEQ ID NO: 13 while the heavy chain having the amino acid sequence represented by SEQ ID NO: 2 may be encoded by a sequence represented by SEQ ID NO: 14, the light chain having the amino acid sequence represented by SEQ ID NO: 3 may be encoded by a sequence represented by SEQ ID NO: 15 while the heavy chain having the amino acid sequence represented by SEQ ID NO: 4 may be encoded by a sequence represented by SEQ ID NO: 16, the light chain having the amino acid sequence represented by SEQ ID NO: 5 may be encoded by a sequence represented by SEQ ID NO: 17 while the heavy chain having the amino acid sequence represented by SEQ ID NO: 6 may be encoded by a sequence represented by SEQ ID NO: 18, the light chain having the amino acid sequence represented by SEQ ID NO: 7 may be encoded by a sequence represented by SEQ ID NO: 19 while the heavy chain having the amino acid sequence represented by SEQ ID NO: 8 may be encoded by a sequence represented by SEQ ID NO: 20, the light chain having the amino acid sequence represented by SEQ ID NO: 9 may be encoded by a sequence represented by SEQ ID NO: 21 while the heavy chain having the amino acid sequence represented by SEQ ID NO: 10 may be encoded with a sequence represented by SEQ ID NO: 22, and the light chain having the amino acid sequence represented by SEQ ID NO: 11 may be encoded by a sequence represented by SEQ ID NO: 23 while the heavy chain having the amino acid sequence represented by SEQ ID NO: 12 may be encoded by a sequence represented by SEQ ID NO: 24.

In addition, the present disclosure provides a pharmaceutical composition for preventing or treating infection by SARS-CoV2, including the antibody as an active ingredient.

The pharmaceutical composition of the present disclosure may be prepared in a unit dose form or prepared by infusion in a multi-dose container through formulation using pharmaceutically acceptable carriers according to a method that may be easily carried out by a person skilled in the art to which the present disclosure pertains.

The pharmaceutically acceptable carriers are those commonly used in preparation, and include lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil, but are not limited thereto. The pharmaceutical composition of the present disclosure may further include lubricants, wetting agents, sweetening agents, flavoring agents, emulsifying agents, suspending agents, and preservatives, in addition to the above components.

In the present disclosure, the content of additives included in the pharmaceutical composition is not particularly limited and may be appropriately adjusted within the content range used for conventional preparation.

The pharmaceutical composition may be formulated in the form of one or more external preparations selected from the group consisting of injectable formulations such as aqueous solutions, suspensions, and emulsions, pills, capsules, granules, tablets, creams, gels, patches, sprays, ointments, emplastrum agents, lotions, liniments, pastas, and cataplasmas.

The pharmaceutical composition of the present disclosure may include pharmaceutically acceptable carriers and diluents, which are additional for formulation. The pharmaceutically acceptable carrier and diluent include excipients such as starch, sugar, and mannitol, fillers and extenders such as calcium phosphate, cellulose derivatives such as carboxymethylcellulose and hydroxypropyl cellulose, binders such as gelatin, alginate, and polyvinylpyrrolidone, lubricants such as talc, calcium stearate, hydrogenated castor oil, and polyethylene glycol, disintegrants such as povidone and crospovidone, and surfactants such as polysorbates, cetyl alcohol, and glycerol, but are not limited thereto. The pharmaceutically acceptable carrier and diluent may be biologically and physiologically compatible with subjects. Examples of the diluent may include saline, aqueous buffers, solvents, and/or dispersion media, but are not limited thereto.

The pharmaceutical composition of the present disclosure may be administered orally or parenterally (e.g., intravenously, subcutaneously, intraperitoneally, or topically) depending on a desired method. For oral administration, the pharmaceutical composition may be formulated as tablets, troches, lozenges, aqueous suspensions, oily suspensions, powder preparation, granules, emulsions, hard capsules, soft capsules, syrups, or elixirs. For parenteral administration, the pharmaceutical composition may be formulated as injections, suppository agents, powder for respiratory inhalation, aerosols for sprays, ointments, powder for application, oil, and creams.

The dosage range of the pharmaceutical composition of the present disclosure may vary depending on the patient's condition, body weight, age, sex, health status, dietary constitution specificity, the nature of preparations, the degree of diseases, administration duration of a composition, administration methods, administration periods or intervals, excretion rate, and drug forms, and may be appropriately selected by those skilled in the art. For example, the dosage may be in the range of about 0.1 to 10,000 mg/kg, but is not limited thereto, and it may be administrated once to several times a day in divided doses.

The pharmaceutical composition may be administered orally or parenterally (e.g., intravenously, subcutaneously, intraperitoneally, or topically) depending on a desired method. A pharmaceutically effective amount and effective dosage of the pharmaceutical composition of the present disclosure may vary depending on formulation methods, administration methods, administration duration, and/or administration routes of the pharmaceutical composition, and those skilled in the art may easily determine and prescribe the dosage effective for desired treatment. Administration of the pharmaceutical composition of the present disclosure may be conducted once a day or several times in divided doses.

### Modes for Carrying Out the Invention

Hereinafter, example embodiments will be described in detail to help the understanding of the present disclosure. However, the following example embodiments are merely illustrative of the content of the present disclosure, and the scope of the present disclosure is not limited to the following example embodiments. The example embodiments of the present disclosure are provided to more completely explain the present disclosure to those skilled in the art.

### <Preparation Example>

Each antibody was derived via solution panning in a human antibody phage library. A day before progression of panning, rhACE2 (ACRO, Human ACE2/ACEH Protein, His Tag, # AC2-H52H8) was conjugated to epoxy beads (Dynabeads M270 epoxy, Thermo #14301). Thereafter, phage library was added to the epoxy beads to which rhACE2 was conjugated, incubation was performed, and then phage library pool that binds to rhACE2 was derived by multiple washing and elution. Thereafter, the derived phage library was divided into single clones, followed by single clone phage ELISA. After coating 96 wells with rhACE2 at a concentration of 0.2 ug/well, each of the single clone phages was treated to each well, and ELISA was performed using secondary antibody (Sino biological, 11973-MM05T-H). The sequences of phage clones binding to rhACE2 in this experiment were identified, and purification into the Sc-Fv-Linker-Fc form was performed.

The amino acid sequence of the prepared antibody was identified via sequencing using vbase2.org. The prepared antibodies were purified using the Sc-Fv-linker-Fc form and the Expi293 system. The DNA sequence of the antibody clones was inserted into pFUSE vector, wherein the pFUSE vector prepared thereby codes information on Sc-Fv-Linker-Fc. Expression of proteins using the pFUSE vector was performed using the Expi293^{™} Expression System Kit (Gibco, A14635). Vector transfection was performed in Expi293 cells with a density of 2∼3×106, and Expifectamine 293 reagent was used for the transfection. Transfected cells were incubated in a shaking incubator for 4 days, and then proteins were harvested.

Purification of proteins was carried out using a high-performance liquid chromatography (HPLC) apparatus (Akta prime, cytiva). HiTrap Protein G HP antibody purification columns (cytiva, # 17040501) were used for purification. The harvested soup was loaded into the column by means of the apparatus, and then antibodies were purified through washing and elution.

The amino acid sequences of each antibody are shown in Table 1 below.

**TABLE 1**

| Types of antibodies | | Amino acid sequence | SEQ ID NO. |
|---|---|---|---|
| C3 | Light chain | QSVLTQPPSVSGAPGQRVTISCTGSSSNIGADYDV HWYQQLPGTAPKLLIYGNTNRPSGVPDRFSGSKS GTSASLAITGLQAEDEANYYCQSYESGLGWLFGG GTQLTVL | 1 |
| | Heavy chain | QVQLVESGGGLVQPGGSLRLSCAASGFTFSSYAM SWVRQAPGKGLEWVSAISGSGGSTYYADSVKGR FTISRDNSKNTLYLQMNSLRAEDTAVYYCAKYGR GLPPGHFDYWGQGTLVTVSS | 2 |
| C8 | Light chain | QSALTQPASASGSPGQSVTISCTGTSSDVGGYNYV SWYQQHPGKAPKLMIYDVSKRPSGVSDRFSGSKS GNTASLTISGLQAEDGADYYCSSYRRGRTVVFGG GTKLTVL | 3 |
| | Heavy chain | QVQLQQWGGGLVQPGGSLRLSCAASGFTFSSYA MSWVRQAPGKGLEWVSAISGSGGSTYYADSVKG RFTISRDNSKNTLYLQMNSLRAEDTAVYYCAKNY FGNPRGFDYWGQGTMVTVSS | 4 |
| C11 | Light chain | ETTLTQSPGTLSVSPGERVTLSCRASQSVSSYLAW YQQKPGQAPRLLIYGASTRATGIPDRFSGSGSGTD FTLTISRLEPEDFAVYYCQQYGISPLNFGGGTKVEI K | 5 |
| | Heavy chain | QVQLVQSGAEVKKPGSSVKVSCKASGGTFSSYAI SWVRQAPGQGLEWMGGVIPIFGTANYAQKFRGR VTMTTDTSTSTAYMELRSLTSDDTAVYYCASPQG TWSTFLEWQGAFSVWGQGTTVTVSS | 6 |
| C14 | Light chain | QAVLTQPSSLSASPGASVSLTCTLRSGINVGAYRV YWYQQKPGSPPQFLLRYKTDSDKQQGSGVPSRFS GSRDASANAGILLISGLRSEDEADYYCAIWHSSA WVFGGGTKVTVL | 7 |
| | Heavy chain | QVQLVQSGAEVKKPGASVKVSCKASGYTFTGYY MHWVRQAPGQGLEWMGRINPNSGGTNYAQKFQ GRVTMTRDTSISTAYMELSRLRSDDTAVYYCARS PVWGSSRNWFDPWGQGTLVTVSS | 8 |
| C21 | Light chain | GIVMTQSPATLSLSPGDRATLSCRASQSVSSSYLA WYQQKPGQAPRLLIYDASNRATGIPARFSGSGSGT DFTLTISSLEPEDFAVYYCQQRSNWPPITFGQGTK LEIK | 9 |
| | Heavy chain | QVQLVESGGGLVQPGGSLRLSCAASGFTFSSYAM SWVRQAPGKGLEWVSAISGSGGSTYYADSVKGR FTISRDNSKDTLYLQMNSLRAEDTAVYYCAKATT VVTPGYFDYWGQGTLVTVSS | 10 |
| C32 | Light chain | DIQMTQSPAIMSASPGERVTMTCSASSSIRYIYWY QQKPGSSPRLLIYDTSNVAPGVPFRFSGSGSGTSYS LTINRMEAEDAATYYCQEWSGYPYTFGGGTKVEI KR | 11 |
| | Heavy chain | QVQLVQSGAEVKKPGSSVKVSCKASGGSFSSYAIS WVRQAPGQGLEWMGGIIPIFGTANYAQKFQGRVT ITADESTSTAYMELSSLRSEDTAVYYCANLRGGSS HYGMDVWGQGTLVTVSS | 12 |

### Example 1. Evaluation on bindability to ACE2

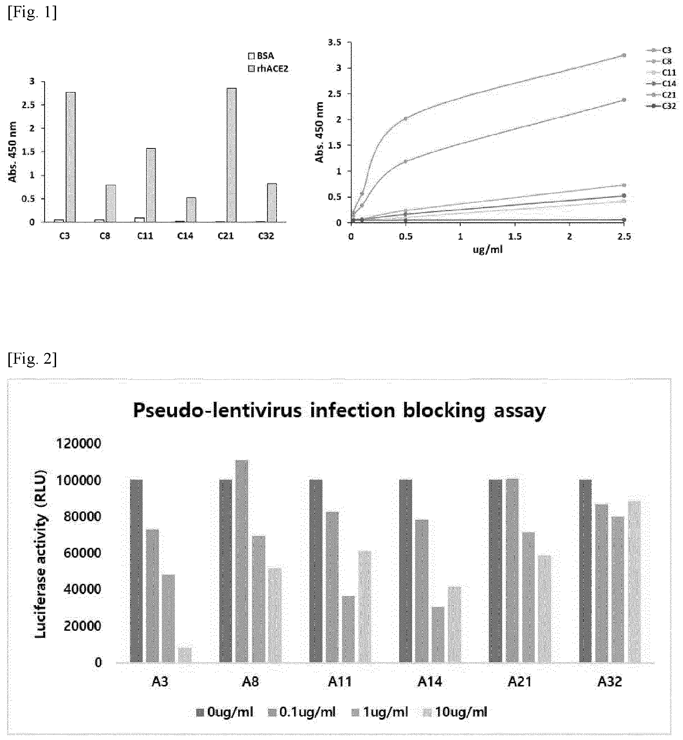

Evaluation on whether the prepared antibody binds to ACE2 was performed. Each antibody was treated, at concentrations of 0.02 ug/ml, 0.1 ug/ml, 0.5 ug/ml, and 2.5 ug/ml, to 96 wells that are coated with rhACE2 at a concentration of 0.2 ug/well, and ELISA was performed using anti-human-Fc-HRP antibody as secondary antibody. A degree of binding was measured with the absorption wavelength at 450 nm. Bovine serum albumin (BSA) was used as a control. As shown in FIG. 1, it was found that all the prepared antibodies bound to rhACE2. In the case of C3 and C21, the degree of binding was higher than that of other antibodies as the concentration increased.

### Example 2. Evaluation on an antiviral effect

In order to determine whether the prepared antibody binds to ACE2 and exhibit an antiviral effect against SARS-CoV2, pseudovirus SARS-2-S was prepared, followed by evaluation on whether the entry thereof into cells is blocked.

The pseudovirus SARS-2-S is a lentivirus that expresses the same spike proteins as SARS-CoV2 and has GFP and luciferase genes inside, making it easy to distinguish whether a cell is infected. Wuhan-Hu-1 Spike-Pseudotyped Lentiviral Kit (NR-52948, BEI resources) was used as a kit to prepare the pseudovirus SARS-2-S. The kit consists of a vector including spike protein (NR-52514, viral entry protein w/ Spike protein), a backbone vector enabling expression of luciferase and GFP (NR-52516, lentiviral backbone w/ Luc2; ZsGreen), and three types of helper plasmids (NR-52517, NR-52518, NR-52519). Five types of plasmids were transfected into HEK293FT cells according to protocol (Crawford KHD. Protocol and Reagents for Pseudotyping Lentiviral Particles with SARS-CoV-2 Spike Protein for Neutralization Assays. Viruses. 2020 May 6;12(5):513. doi: 10.3390/v12050513. PMID: 32384820; PMCID: PMC7291041.) provided along with a product, incubation was performed, and then the pseudovirus was harvested.

HEK-293T-hACE2 cells (NR-52511, BEI resources) were pretreated with the prepared antiboty at concentrations of 0 ug/ml, 0.1 ug/ml, 1 ug/ml, or 10 ug/ml at 37°C in the presence of 5% CO₂ for 1 hour. Then, in the presence of antibody, the pseudovirus SARS-2-S (SARS-related coronavirus 2) was infected using the Wuhan-Hu-1 Spike-Pseudotyped Lentiviral Kit (NR-52948, BEI resources). Cells were lysed at 48 hpi, luciferase activity was measured with Bright-Glo^{™} (E2610, Promega, Madison, WI, USA) according to the manufacturer's instructions, and luciferase activity was quantified using a Perkinelmer EnVision microplate reader.

As shown in FIG. 2, it was found that the degree of infection by SARS-2-S decreased as the pretreatment concentration of the prepared antibody increased. The results demonstrate that the antibodies exhibit an antiviral effect to prevent infection by SARS-CoV2 through the binding of ACE2.

As described above, a specific part of the content of the present disclosure is described in detail, for those of ordinary skill in the art, it is clear that the specific description is only a preferred embodiment, and the scope of the present disclosure is not limited thereby. In other words, the substantial scope of the present disclosure may be defined by the appended claims and their equivalents.

## Claims

1. An antiviral antibody against SARS-CoV2 binding to ACE2, comprising: a light chain having an amino acid sequence represented by SEQ ID NO: 1; and a heavy chain having an amino acid sequence represented by SEQ ID NO: 2.

2. An antiviral antibody against SARS-CoV2 binding to ACE2, comprising: a light chain having an amino acid sequence represented by SEQ ID NO: 3; and a heavy chain having an amino acid sequence represented by SEQ ID NO: 4.

3. An antiviral antibody against SARS-CoV2 binding to ACE2, comprising: a light chain having an amino acid sequence represented by SEQ ID NO: 5; and a heavy chain having an amino acid sequence represented by SEQ ID NO: 6.

4. An antiviral antibody against SARS-CoV2 binding to ACE2, comprising: a light chain having an amino acid sequence represented by SEQ ID NO: 7; and a heavy chain having an amino acid sequence represented by SEQ ID NO: 8.

5. An antiviral antibody against SARS-CoV2 binding to ACE2, comprising: a light chain having an amino acid sequence represented by SEQ ID NO: 9; and a heavy chain having an amino acid sequence represented by SEQ ID NO: 10.

6. An antiviral antibody against SARS-CoV2 binding to ACE2, comprising: a light chain having an amino acid sequence represented by SEQ ID NO: 11; and a heavy chain having an amino acid sequence represented by SEQ ID NO: 12.

7. A pharmaceutical composition for preventing or treating infection by SARS-CoV2, comprising the antibody in any one of claims 1 to 6 as an active ingredient.
